# EUROPÄISCHE PATENTSCHRIFT

(11) **EP 0 661 541 B1**
(45) Veröffentlichungstag und Bekanntmachung des Hinweises auf die Patenterteilung: **01.12.1999**
(21) Anmeldenummer: 94120089.1
(22) Anmeldetag: 19.12.1994
(51) Int. Cl.: G01N 33/96, G01N 33/53

(54) **Verfahren zur Herstellung langzeitstabiler klarer Seren**
Method of producing long time stable, clear serums
Procédé de fabrication de serums clairs et stables à longue durée

(30) Priorität: 29.12.1993 DE 4344868
(43) Veröffentlichungstag der Anmeldung: 05.07.1995
(73) Patentinhaber: Dade Behring Marburg GmbH, 35001 Marburg (DE)
(72) Erfinder: Vermeer, Hans, Dr., D-35094 Lahntal-Gossfelden (DE); Toth, Tibor, Dr., D-35041 Marburg (DE); Münscher, Gerhard, Dr., D-35041 Marburg (DE)

(56) Entgegenhaltungen:
- EP-A- 0 294 714
- EP-A- 0 554 657
- DE-A- 3 303 083

## Beschreibung

Die vorliegende Erfindung betrifft ein Verfahren zur Herstellung langzeitstabiler klarer Seren sowie deren Verwendung in der Diagnostik.

Immunologische Diagnose-Methoden werden wegen ihrer hohen Spezifität, Empfindlichkeit und Schnelligkeit in zunehmenden Maße angewendet. Von diesen Methoden haben die Präzipitationsmethoden zur quantitativen Bestimmung von Serum-Proteinen (radiale Immundiffusion, Nephelometrie, Turbidimetrie) einen besonders hohen Stellenwert. Seit einigen Jahren haben nephelometrische und turbidimetrische Bestimmungsmethoden wegen ihrer hohen Präzision, Schnelligkeit und Automatisierbarkeit zunehmend Zugang zu medizinischen Untersuchungs-Laboratorien gefunden. Diese Methoden machen von der Eigenschaft von Antigenen und/oder Antikörpern Gebrauch, mit dem entsprechenden Partner in einer immunchemischen Reaktion Immunkomplexe zu bilden. Die nach Mischung beider Partner einsetzende Bildung von Antigen-Antikörper-Komplexen kann dann z. B. photometrisch gemessen werden.

Die quantitative Bestimmung der Serum-Proteine liefert wichtige Hinweise zur Diagnose und zur Beurteilung von Krankheitsverläufen.

An die Antisera, die für die Diagnostik, z. B. von Serumproteinen verwendet werden, werden sehr hohe Anforderungen gestellt. Sie müssen neben einer hohen Konzentration an spezifischen Antikörpern, gute Stabilität bei verschiedenen Temperaturen und hohe Lichtdurchlässigkeit aufweisen. Gleiches gilt für Standard- und Kontroll-Seren. Im folgenden werden alle diese Seren zusammenfassend als "Seren" bezeichnet.

Eine Schwierigkeit, die bei der Herstellung solcher Seren auftritt, besteht darin, daß sie labile Komponenten enthalten, die bei der Herstellung soweit möglich entfernt werden müssen, da sonst Trübungen und Flocken entstehen können, die dann vor der Anwendung filtriert oder zentrifugiert werden müssen.

Für die Beseitigung von Trübungen in Serum-Proben wurden bisher verschiedene Methoden vorgeschlagen, die aber insbesondere bei tierischen Antiseren nur von eingeschränktem Nutzen sind.

So ist z. B. in Clin. Chem. 29, 120 - 125 (1983) ein Verfahren beschrieben, bei dem Serumtrübungen durch Ausschütteln mit einem Gemisch organischer Lösungsmittel entfernt werden. Diese Methode erfordert einen zusätzlichen Verfahrensschritt, bei dem es darüber hinaus insbesondere bei stark lipämischen Seren u.a. zu unkontrollierbaren Volumenveränderungen des Probenmaterials kommen kann und damit zu einer Verfälschung der Meßergebnisse. Schließlich ist bei Einsatz dieses Verfahrens auch nicht mehr die Bestimmung von Serumbestandteilen möglich, die bei der Extraktion ganz oder teilweise entfemt werden.

Ein weiteres Problem ist die Entsorgung des Extraktionsmittels, insbesondere, wenn es sich um einen Halogen-Kohlenwasserstoff handelt.

Zur Adsorption der Lipoproteine wurden auch eine Reihe von selektiv bindenden Adsorbentien eingesetzt, deren gemeinsames Merkmal eine hydrophile Matrix ist, an die Phenyl- oder Alkyl-Gruppen gebunden sind. Weiterhin wurden Lipoprotein-bindende Adsorbentien auf Basis von Siliziumdioxyd, die z. B. unter der Bezeichnung Aerosil® im Handel sind, beschrieben.

In der EP 0 137 221 wird die Entfernung von Lipoproteinen mittels Polyhydroxymethylen beschrieben. Auch Polyanionen wie Dextransulfat oder Heparin können zur Ausfällung in Gegenwart von Kationen (Mg²⁺, Mn²⁺) verwendet werden. Nachteilig ist, daß im Überstand verbliebene Mengen an Polyanionen und Metallionen für die meisten Anwendungen entfernt werden müssen.

In EP 0 294 714 wird die Verwendung von ca. 0,3 Gew. % Polyvinylpyrrolidon zur Vermeidung der Trübung lyophilisierter Kontroll-Sera nach Wiederauflösen beschrieben.

EP 0 554 657 betrifft ein immunochemisches Verfahren zum Nachweis eines Partners einer Antigen-Antikörper-Reaktion in Gegenwart künstlicher Immunkomplexe. Damit die Immunkomplexe in Lösung stabilisiert bleiben werden sie bevorzugt in Gegenwart eines zyklischen Amids, wie Pyrrolidon, hergestellt.

Zur Vermeidung von Störungen bei immunchemischer Bestimmung eines Serumproteins wird in der DE-OS 28 29 531 ein kationisches Tensid verwendet.

In EP 0 130 537 werden zu einer biologischen Flüssigkeit (z.B. Serum) zur Beseitigung von Trübungen oberflächenaktive Mittel verwendet, welche aus einem polyethoxylierten Triglycerid und n-Alkansulfonat sowie ggf. einem nicht - oder anionischen Tensid bestehen.

Die geschilderten Verfahren können zwar bei Trübungen, die durch Chylomikronen und VLDL (Very Low Density Lipoprotein) hervorgerufen wurden, eine gewisse Abhilfe bei der Durchführung von photometrischen Analysen schaffen, es hat sich aber gezeigt, daß es insbesondere bei längerer Lagerung (≥ 1 Jahr) immer wieder zu Trübungen und Ausflockungen kommt.

Die Ursachen für diese Trübungen können sehr vielfältig sein, sie werden offensichtlich nur teilweise von Lipiden und Lipoproteinen verursacht. Weitere Ursachen könnten z. B. die während der Immunisierung entstandenen Immunkomplexe sein.

Aufgabe der Erfindung war es deshalb, ein einfaches Verfahren und/oder Mittel für die Herstellung von langzeitstabilen klaren Seren insbesondere für die immunchemische Protein-Bestimmung zu finden.

Überraschenderweise wurde gefunden, daß die bekannten Nachteile überwunden werden können, wenn die Seren mit einer Verbindung der allgemeinen Formel (I) versetzt werden 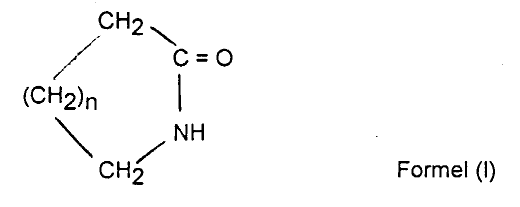 n = 0 - 8, bevorzugterweise n = 1 bis 4 ist.

Diese an sich dem Fachmann bekannten Verbindungen gehören den cyclischen Carbonsäure-Amiden an und zeichnen sich durch ein hohes Dipolmoment und sehr gute Lösungseigenschaften aus. Bevorzugte Vertreter dieser Verbindungsklasse sind Butyrolactam (Pyrrolidon), Valerolactam und Caprolactam.

Gegenstand dieser Erfindung ist also ein Verfahren zur Herstellung langzeitstabiler klarer Seren, die in der Diagnostik verwendet werden, wobei den Seren eine zur Verhinderung und/oder Beseitigung einer Trübung ausreichende Menge einer Verbindung der Formel (I) zugesetzt wird.

Die Verbindungen können allein oder in Kombinationen verwendet werden. Besonders vorteilhaft ist die Kombination von Butyrolactam/Caprolactam.

Die erfindungsgemäßen Verbindungen werden meist in Konzentrationen von 0,2 - 30 Gew. % verwendet, vorzugsweise 1 - 10 Gew. %, besonders bevorzugterweise von 1,5-3 Gew. %.

Es kann auch zweckmäßig sein, die erfindungsgemäßen Verbindungen zunächst in einer geeigneten wäßrigen Lösung zu lösen und dann den Seren zuzusetzen. Solche wässrige Lösungen sind dem Fachmann an sich bekannt, geeignet sind z. B. isotonische Kochsalzlösung oder 0,15 molare Glycin-NaCl-Pufferlösung, pH 7,2 bis pH 10, vorzugsweise etwa pH 8,0. Auch Tris-, Phosphat-, Borat-lmidazol- oder Acetat-Puffer-Lösungen sind geeignet.

Seren im Sinne der Erfindung sind auch Fraktionen, die bei der Aufreinigung von Seren anfallen, insbesondere auch Gamma-Globulin Fraktionen.

Besonders geeignet sind die erfindungsgemäßen Verbindungen für die Stabilisierung von tierischen Antisera gegen Humanproteine, wie z. B. Anti-Human-lgG (vom Kaninchen), Anti-α₂-Makroglobulin (vom Kaninchen), Anti-Human-lgM (von der Ziege), Anti-Human-Albumin (vom Schaf), Anti-α₁-Antitrypsin (vom Kaninchen), Anti-Human-Apolipoprotein A-l (vom Kaninchen), Anti-Human-Apolipoprotein B (vom Kaninchen) oder für Standard- und Kontroll-Sera humanen Ursprungs, die z. B. Rheumafaktoren und/oder Immunkomplexe enthalten können.

Die erfindungsgemäßen Verbindungen werden bevorzugterweise einem weitgehend nativen Antiserum in einer solchen Menge zugegeben, daß die Konzentration 0.2 - 30 Gew. % beträgt. Zusätzlich können auch noch andere bekannte Stabilisatorsubstanzen, wie z. B. Natriumazid oder Antibiotika zugegeben werden. Bevorzugterweise wird das Serum nach der Zugabe der erfindungsgemäßen Verbindungen sterilfiltriert und steril abgefüllt.

Gegenstand dieser Erfindung ist ferner ein Serum zur Verwendung in der Diagnostik dadurch gekennzeichnet, daß es eine zur Verhinderung und/oder Beseitigung von Trübungen wirksame Menge einer oder mehrerer Verbindungen der Formel (I) enthält.

Gegenstand dieser Erfindung ist außerdem die Verwendung der erfindungsgemäßen Seren als Standard- und/oder Kontrollserum in einem diagnostischen Verfahren. Vorteilhaft ist die Verwendung der erfindungsgemäßen Seren in diagnostischen Verfahren, die auf dem Prinzip der Immunpräzipitation beruhen, wie z. B. die radiale Immundiffusion, die Nephelometrie und die Turbidimetrie.

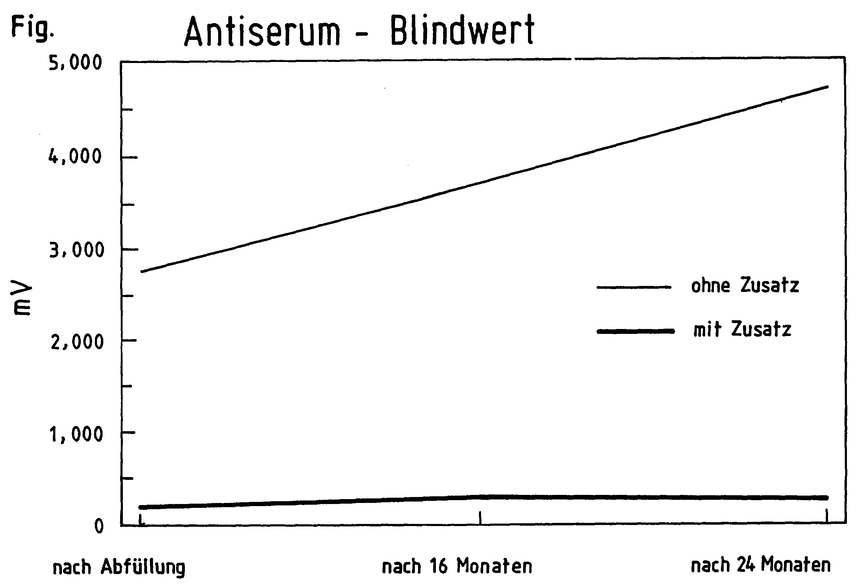

Wie unter anderem aus der Figur hervorgeht, weist das Reagenz ohne den erfindungsgemäßen Zusatz bereits nach der Abfüllung einen hohen Blindwert auf, der im Verlaufe der Lagerung noch weiter zunimmt, während das Reagenz mit dem erfindungsgemäßen Zusatz klar ist und nur einen geringfügigen Anstieg des Blindwertes zeigt. Es zeigt sich außerdem ein gewisser stabilisierender Effekt des erfindungsgemäßen Zusatzes, wie aus der Tab. 1 entnommen werden kann.

Als Maß für eine Trübung kann entweder ein Durchlichtsignal oder ein Streulichtsignal verwendet werden. Bevorzugterweise steigt die Trübung in dem erfindungsgemäßen Reagenz in einem Lagerungszeitraum von 12 Monaten um weniger als 50 % an. Als optisch klar wird z. B. ein Serum bezeichnet, das unter den in den Beispielen verwendeten Bedingungen ein Streulichtsignal von weniger als 1000, bevorzugterweise weniger als 600, besonders bevorzugterweise weniger als 300 mV aufweist.

Langzeitstabil im Sinne der Erfindung heißt, daß das Serum bei Lagerung vorteilhafterweise bei 2 - 8 °C über einen Zeitraum von 12 Monaten, bevorzugterweise 24 Monaten optisch klar bleibt.

Die folgenden Beispiele erläutern die Erfindung, schränken sie aber in keiner Weise ein.

### Beispiel 1

a) 300 ml Antiserum gegen Human-Apolipoprotein A-I (vom Kaninchen) wurden mit 1,5 Vol. % 2-Pyrrolidon (γ-Butyrolactam) und 0,285 g Natriumazid versetzt und sterilfiltriert. Nach Sterilfiltration wurde das Antiserum unter sterilen Bedingungen zu 5 ml abgefüllt. Der Verlauf der Standardkurve veränderte sich nach Lagerung des Antiserums über einen Zeitraum von 7, 16 und 24 Monaten bei +2 bis +8 °C nicht. Alle Antiserum-Abfüllungen waren klar.
b) 300 ml Antiserum gegen Human-Apolipoprotein A-I (vom Kaninchen) wurden wie unter Beispiel 1 a) beschrieben, jedoch ohne 2-Pyrrolidon (γ-Butyrolactam), sterilfiltriert, abgefüllt und bei +2 bis +8 °C gelagert. Das Antiserum zeigte bereits nach 7 Monaten in allen Flaschen Trübungen und eine nephelometrische Apolipoprotein A-I-Bestimmung war ohne vorherige Filtration nicht möglich.

Die Prüfung der Seren erfolgte durch nephelometrische Messung einer Standard-Reihe. Dazu wurde ein Standard-Serum mit 1 620 mg/l Apolipoprotein A-I eingesetzt; die Standard-Reihe am Gerät automatisch 1 : 5 bis 1 : 160 verdünnt, d. h. es wurden Konzentrationen von 324 bis 10 mg/l erhalten. Zur Messung wurden 10 µl Standard-Verdünnung mit 40 µl Antiserum gegen Human-Apolipoprotein A-I eingesetzt.

**Tab. 1**

| | **Streulichtsignal** (Bit) | | | | | |
|---|---|---|---|---|---|---|
| **Standard-Verdünnung** | nach Abfüllung | | nach 16 Monaten | | nach 24 Monaten | |
| | ohne Zusatz | mit Zusatz | ohne Zusatz - nach Filtration | mit Zusatz - ohne Filtration | ohne Zusatz - nach Filtration | mit Zusatz - ohne Filtration |
| 1 : 5 | 1 589 | 1 560 | 1 475 | 1 377 | 1 452 | 1 502 |
| 1 : 10 | 1 074 | 1 067 | 1 098 | 1 096 | 982 | 1 036 |
| 1 : 20 | 593 | 589 | 622 | 623 | 546 | 571 |
| 1 : 40 | 287 | 279 | 274 | 304 | 260 | 272 |
| 1: 80 | 129 | 119 | 92 | 126 | 104 | 114 |
| 1 : 160 | 52 | 42 | 22 | 49 | 22 | 46 |
| **Leerwert** | 2 | 2 | 13 | 0 | -1 | 2 |
| " | 2 | 0 | 8 | 3 | 0 | 2 |
| " | 0 | -1 | -4 | 0 | 11 | 0 |
| **AS-Blindwert** mV | 2 750 | 160 | 3 700 | 280 | 4 700 | 270 |
| opt. Kontr. | Trübungen | klar | Trübungen | klar | Trübungen | klar |

Die Messung des AS-Blindwertes erfolgte mit dem Behring Laser-Nephelometer

### Beispiel 2

a) 500 ml Antiserum gegen Human-lgG (vom Kaninchen) wurden mit 3,0 Vol. % 2-Pyrrolidon (γ-Butyrolactam) und 0,475 g Natriumazid versetzt und sterilfiltriert. Nach Sterilfiltration wurde das Antiserum unter sterilen Bedingungen zu 5 ml abgefüllt. Der Verlauf der Standardkurve veränderte sich nach Lagerung des Antiserums über einen Zeitraum von 28 Monaten bei +2 bis +8 °C nicht. Alle Antiserum-Abfüllungen waren klar.
b) 500 ml Antiserum gegen Human-lgG (vom Kaninchen) wurden wie unter Beispiel 2 a) beschrieben, jedoch ohne 2-Pyrrolidon (γ-Butyrolactam), sterilfiltriert, abgefüllt und bei +2 bis +8 °C gelagert. Das Antiserum zeigte nach 2 Jahren in allen Flaschen Trübungen und Ausflockungen und war für die nephelometrische IgG-Bestimmung ohne erneute Filtration nicht mehr geeignet.

Die Prüfung der Seren erfolgte durch nephelometrische Messung einer Standard-Reihe. Dazu wurde ein Standard-Serum mit 12 000 mg/l IgG eingesetzt; die Standard-Reihe am Gerät automatisch 1 : 80 bis 1 : 2 560 verdünnt, d. h. es wurden Konzentrationen von 150 bis 4,6 mg/l erhalten. Zur Messung wurden 100 µl Standard-Verdünnung mit 40 µl Antiserum gegen·Human-IgG eingesetzt.

**Tab. 2**

| | **Streulichtsignal** (Bit) | | | |
|---|---|---|---|---|
| **Standard-Verdünnung** | nach Abfüllung | | nach 24 Monaten | |
| | ohne Zusatz | mit Zusatz | ohne Zusatz - nach Filtration | mit Zusatz - ohne Filtration |
| 1: 80 | 1 122 | 1 113 | 1 071 | 1 105 |
| 1: 160 | 802 | 774 | 782 | 758 |
| 1: 320 | 520 | 506 | 511 | 478 |
| 1: 640 | 300 | 283 | 295 | 260 |
| 1 : 1 280 | 146 | 129 | 130 | 126 |
| 1 : 2 560 | 64 | 54 | 47 | 52 |
| **Leerwert** | 0 | 2 | 4 | 1 |
| " | 0 | 2 | 1 | 3 |
| " | -1 | 2 | 3 | 0 |
| **AS-Blindwert** mV | 600 | 270 | 1 600 | 340 |
| opt. Kontr. | klar | klar | Trübungen | klar |

Die Messung des AS-Blindwertes erfolgte mit dem Behring Laser-Nephelometer

### Beispiel 3

a) 100 ml Antiserum gegen Human-α₁-Antitrypsin (vom Kaninchen) wurden mit 1,5 Vol. % 2-Pyrrolidon (γ-Butyrolactam) und 0,095 g Natriumazid versetzt und sterilfiltriert. Nach Sterilfiltration wurde das Antiserum unter sterilen Bedingungen zu 5 ml abgefüllt. Der Verlauf der Standardkurve veränderte sich nach Lagerung des Antiserums über einen Zeitraum von 1 Monat bei +2 bis +8 °C nicht. Alle Antiserum-Abfüllungen waren klar.
b) 100 ml Antiserum gegen Human-α₁-Antitrypsin (vom Kaninchen) wurden wie unter Beispiel 3 a) beschrieben, jedoch ohne 2-Pyrrolidon (γ-Butyrolactam), sterilfiltriert, abgefüllt und bei +2 bis +8 °C gelagert. Das Antiserum zeigte bereits nach 1 Monat in allen Flaschen Trübungen und Ausflockungen. Es war für die nephelometrische α₁-Antitrypsin-Bestimmung erst nach erneuter Filtration geeignet.

Die Prüfung der Seren erfolgte durch nephelometrische Messung einer Standard-Reihe. Dazu wurde ein Standard-Serum mit 1 650 mg/l α₁-Antitrypsin eingesetzt; die Standard-Reihe am Gerät automatisch 1 : 5 bis 1 : 160 verdünnt, d. h. es wurden Konzentrationen von 330 bis 10,3 mg/l erhalten. Zur Messung wurden 20 µl Standard-Verdünnung mit 40 µl Antiserum gegen Human-α₁-Antitrypsin eingesetzt.

**Tab. 3**

| | **Streulichtsignal** (Bit) | | | |
|---|---|---|---|---|
| **Standard-Verdünnung** | nach Abfüllung | | nach 1 Monat | |
| | ohne Zusatz | mit Zusatz | ohne Zusatz - nach Filtration | mit Zusatz - ohne Filtration |
| 1 : 5 | 1 490 | 1 410 | 1 416 | 1 456 |
| 1: 10 | 993 | 983 | 957 | 981 |
| 1: 20 | 608 | 591 | 601 | 598 |
| 1: 40 | 306 | 318 | 321 | 297 |
| 1 : 80 | 133 | 130 | 134 | 130 |
| 1 : 160 | 48 | 45 | 57 | 46 |
| **Leerwert** | 0 | 0 | 10 | 1 |
| " | 0 | 0 | 12 | 1 |
| " | 0 | 0 | 1 | 1 |
| **AS-Blindwert** | | | | |
| mV | 160 | 120 | 5 430 | 250 |
| Bit | 7 | 16 | 412 | 13 |
| opt. Kontr. | klar | klar | Trübungen | klar |

Die Messung des AS-Blindwertes erfolgte mit dem Behring Laser-Nephelometer

### Beispiel 4

a) 100 ml Antiserum gegen Human-α₁-Antitrypsin (vom Kaninchen) wurden mit 1,5 Vol. % 2-Pyrrolidon (γ-Butyrolactam) und 0,095 g Natriumazid versetzt und sterilfiltriert. Nach Sterilfiltration wurde das Antiserum unter sterilen Bedingungen zu 5 ml abgefüllt. Der Verlauf der Standardkurve veränderte sich nach Lagerung des Antiserums über einen Zeitraum von 6 Monaten bei +2 bis +8 °C nicht. Alle Antiserum-Abfüllungen waren klar.
b) 100 ml Antiserum gegen Human-α₁-Antitrypsin (vom Kaninchen) wurden wie unter Beispiel 4 a) beschrieben, jedoch ohne 2-Pyrrolidon (γ-Butyrolactam), sterilfiltriert, abgefüllt und bei +2 bis +8 °C gelagert. Das Antiserum zeigte bereits nach 6 Monaten in allen Flaschen Trübungen und Ausflockungen. Die Standardkurve war für die α₁-Antitrypsin-Bestimmung unbrauchbar.

Die Prüfung der Seren erfolgte durch nephelometrische Messung einer Standard-Reihe. Dazu wurde ein Standard-Serum mit 2 000 mg/l α₁-Antitrypsin eingesetzt; die Standard-Reihe am Gerät automatisch 1 : 5 bis 1 : 160 verdünnt, d. h. es wurden Konzentrationen von 400 bis 12,5 mg/l erhalten. Zur Messung wurden 20 µl Standard-Verdünnung mit 40 µl Antiserum gegen Human-α₁-Antitrypsin eingesetzt.

**Tab. 4.**

| | **Streulichtsignal** (Bit) | | | |
|---|---|---|---|---|
| **Standard-Verdünnung** | nach Abfüllung | | nach 6 Monaten | |
| | ohne Zusatz | mit Zusatz | ohne Zusatz - nach Filtration | mit Zusatz - ohne Filtration |
| 1 : 5 | 1 670 | 1 705 | 1 685 | 1 806 |
| 1 : 10 | 1 264 | 1 276 | 1 214 | 1 350 |
| 1 : 20 | 841 | 855 | 817 | 887 |
| 1 : 40 | 474 | 507 | 471 | 513 |
| 1 : 80 | 241 | 255 | 232 | 279 |
| 1 : 160 | 113 | 126 | 125 | 128 |
| **Leerwert** | 0 | 2 | 57 | 14 |
| " | 3 | 6 | 48 | 9 |
| " | 2 | 8 | ..... 47 | 11 |
| **AS-Blindwert** | | | | |
| mV | 60 | 100 | 11 140 | 280 |
| opt. Kontr. | klar | klar | Trübungen | klar |

Die Messung des AS-Blindwertes erfolgte mit dem Behring Laser-Nephelometer

### Beispiel 5

100 ml Antiserum gegen Human-α₁-Antitrypsin (vom Kaninchen) wurden mit 1,5 % δ-Valerolactam und 0,095 g Natriumazid versetzt, sterilfiltriert, abgefüllt und bei +2 bis +8 °C gelagert. Das Antiserum zeigte nach 6 Monaten ohne Zusatz des erfindungsgemäßen Mittels massive Ausflockungen, während die mit δ-Valerolactam (hergestellt nach der Erfindung) versetzte Präparation vollkommen klar und ohne Ausflockungen bzw. Trübungen war.

### Beispiel 6

a) 100 ml Antiserum gegen Human-Präalbumin (vom Kaninchen) wurden mit 1,5 Vol. % 2-Pyrrolidon (γ-Butyrolactam) und 0,095 g Natriumazid versetzt und sterilfiltriert. Nach Sterilfiltration wurde das Antiserum unter sterilen Bedingungen zu 2 ml abgefüllt. Der Verlauf der Standardkurve veränderte sich nach Lagerung des Antiserums über einen Zeitraum von 1 Monat bei +2 bis +8 °C nicht. Alle Abfüllungen waren vollkommen klar und ohne Ausflockungen.
b) 100 ml Antiserum gegen Human-Präalbumin (vom Kaninchen) wurden wie unter Beispiel 6 a) beschrieben, jedoch ohne 2-Pyrrolidon (γ-Butyrolactam), sterilfiltriert, abgefüllt und bei +2 bis +8 °C gelagert. Das Antiserum zeigte bereits nach 1 Monat in allen Flaschen erhebliche Trübungen. Erst nach dem Entfernen der Ausflockungen durch Filtration konnte das Antiserum für die Präalbumin-Bestimmung eingesetzt werden.

Die Prüfung der Seren erfolgte durch nephelometrische Messung einer Standard-Reihe. Dazu wurde ein Standard-Serum mit 310 mg/l Präalbumin eingesetzt; die Standard-Reihe am Gerät automatisch 1 : 2,5 bis 1 : 80 verdünnt, d. h. es wurden Konzentrationen von 124 bis 3,9 mg/l erhalten. Zur Messung wurden 50 µl Standard-Verdünnung mit 40 µl Antiserum gegen Human-Präalbumin eingesetzt.

**Tab. 5**

| | **Streulichtsignal** (Bit) | | | |
|---|---|---|---|---|
| **Standard-Verdünnung** | nach Abfüllung | | nach 2 Monaten | |
| | ohne Zusatz | mit Zusatz | ohne Zusatz - nach Filtration | mit Zusatz - ohne Filtration |
| 1 : 2,5 | 1 283 | 1 257 | 1 275 | 1 278 |
| 1 : 5 | 995 | 970 | 978 | 975 |
| 1:10 | 620 | 612 | 612 | 615 |
| 1 :20 | 339 | 332 | 347 | 335 |
| 1 :40 | 183 | 167 | 189 | 168 |
| 1 :80 | 84 | 71 | 95 | 78 |
| **Leerwert** | 0 | 3 | 10 | 0 |
| " | -1 | 0 | 12 | 1 |
| " | 2 | 1 | 9 | 3 |
| **AS-Blindwert** | | | | |
| mV | 120 | 150 | 2 810 | 200 |
| Bit | 16 | 5 | 389 | 6 |
| opt. Kontr. | klar | klar | Trübungen | klar |

Die Messung des AS-Blindwertes erfolgte mit dem Behring Laser-Nephelometer

### Beispiel 7

a) 500 ml Antiserum gegen Human-Apolipoprotein B (vom Kaninchen) wurden mit 3,0 Vol. % 2-Pyrrolidon (γ-Butyrolactam) und 0,475 g Natriumazid versetzt und sterilfiltriert. Nach Abfüllung zu 5 ml wurden die Proben bei +2 bis +8 °C gelagert. Nach 3 Jahren zeigten die Proben weder Trübungen noch Ausflockungen.
b) 47 710 ml Antiserum gegen Human-Apolipoprotein B (vom Kaninchen) wurden mit 45,3 g Natriumazid versetzt, sterilfiltriert und abgefüllt zu 5 ml. Die bei +2 bis +8 °C gelagerten Abfüllungen zeigten nach 30 Monaten Trübungen und Flokken.

### Beispiel 8

### Herstellung eines Kontroll-Serums (human) für Rheumafaktoren, Anti-Streptolysin 0 und CRP

Je 50 ml Rheumafaktoren positives Serum (human) von 4 verschiedenen Spendern wurden gemischt. 19 ml dieses Serumpools mit einem Gehalt von 674 lU/ml RF wurden mit 81 ml isotonischer Kochsalzlösung und 100 ml einer 1,8%igen Lösung von Human-Gamma-Globulin mit einem Gehalt von ca. 1 800 lU/ml ASL (Reinheitsgrad mind. 95 %) verdünnt. Anschließend wurden 10 mg/ml CRP, 0,2 g Natriumazid, 5 ml Butyrolactam und 0,4 g Benzamidiniumchlorid zugesetzt. Nach Sterilfiltration und Abfüllung wurden Proben bei +2 bis +8 °C, bei Raumtemperatur und bei +37 °C gelagert.

Das Kontroll-Serum zeigte ohne Zusatz von Butyrolactam bereits nach 2 Wochen Ausflockungen, während die erfindungsgemäß mit Butyrolactam versetzten Abfüllungen nach 6 Monaten vollkommen klar und ohne Ausflockungen waren.

### Beispiel 9

a) 500 ml Antiserum gegen Human-α₂-Makroglobulin (vom Kaninchen) wurden mit 3,0 Vol. % 2-Pyrrolidon (γ-Butyrolactam) und 0,475 g Natriumazid versetzt und sterilfiltriert. Nach Abfüllung zu 5 ml wurden die Flaschen bei +2 bis +8 °C gelagert. Nach 2, 5, 17 und 26 Monaten sind die Abfüllungen vollkommen klar.
b) 500 ml Antiserum gegen Human-α₂-Makroglobulin (vom Kaninchen) wurden mit 0,475 g Natriumazid versetzt und sterilfiltriert. Die wie im Beispiel 7 a) gelagerten Abfüllungen zeigten bereits 9 Wochen nach Sterilfiltration und Abfüllung Trübungen und Flocken.

Die Prüfung der Seren erfolgte durch nephelometrische Messung einer Standard-Reihe. Dazu wurde ein Standard-Serum mit 1 790 mg/l α₂-Makroglobulin eingesetzt; die Standard-Reihe am Gerät automatisch 1 : 5 bis 1 : 160 verdünnt, d. h. es wurden Konzentrationen von 358 bis 11,2 mg/l erhalten. Zur Messung wurden 20 µl Standard-Verdünnung mit 40 µl Antiserum gegen Human-α₂-Makroglobulin eingesetzt.

**Tab. 6**

| | **Streulichtsignal** (Bit) | | | | | |
|---|---|---|---|---|---|---|
| **Standard-Verdünnung** | nach Abfüllung | | nach 6 Monaten | | nach 18 Monaten | |
| | ohne Zusatz | mit Zusatz | ohne Zusatz - nach Filtration | mit Zusatz - ohne Filtration | ohne Zusatz - nach Filtration | mit Zusatz - ohne Filtration |
| 1: 5 | 1 208 | 1 141 | 1 300 | 1 190 | 1 304 | 1 205 |
| 1: 10 | 739 | 687 | 844 | 772 | 836 | 733 |
| 1: 20 | 421 | 373 | 446 | 421 | 488 | 419 |
| 1: 40 | 198 | 172 | 230 | 196 | 248 | 186 |
| 1: 80 | 95 | 75 | 91 | 86 | 111 | 85 |
| 1: 160 | 47 | 31 | 45 | 38 | 38 | 33 |
| **Leerwert** | 6 | 1 | 1 | - 6 | 7 | 1 |
| " | 5 | 0 | 1 | 7 | 0 | 0 |
| " | 16 | 0 | 2 | 4 | 0 | 0 |
| **AS-Blindwert** | | | | | | |
| mV | 1 400 | 140 | 7 460 | 170 | 3 860 | 190 |
| | | | | | 90 | 27 |
| opt. Kontr. | klar | klar | Trübungen | klar | Trübungen | klar |

Die Messung des AS-Blindwertes erfolgte mit dem Behring Laser-Nephelometer

## Patentansprüche

1. Verfahren zur Herstellung langzeitstabiler klarer Seren, die in der Diagnostik verwendet werden, dadurch gekennzeichnet, daß den Seren eine zur Verhinderung und/oder Beseitigung einer Trübung, ausreichende Menge mindestens einer Verbindung der Formel (I) zugesetzt wird, 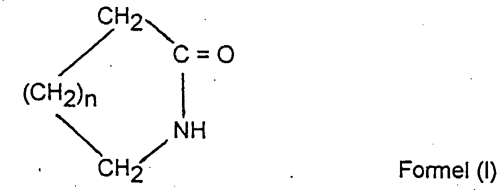 worin n = 0 - 8, bevorzugterweise 1 - 4.

2. Verfahren nach Anspruch 1, dadurch gekennzeichnet, daß man Verbindungen der Formel (I) den Seren in solchen Mengen zusetzt, daß die Konzentration die ser Verbindungen im Serum 0,2 bis 30 Gew. % beträgt.

3. Verfahren nach den Ansprüchen 1 und 2 dadurch gekennzeichnet, daß die Verbindung der Formel (I) Butyrolactam ist.

4. Verfahren nach den Ansprüchen 1 bis 3 dadurch gekennzeichnet, daß eine Mischung aus mindestens 2 der Verbindungen der Formel (I) zugesetzt wird.

5. Verfahren nach Anspruch 4 dadurch gekennzeichnet, daß Butyrolactam und Caprolactam zugesetzt werden.

6. Verfahren nach mindestens einem der Ansprüche 1 bis 5 dadurch gekennzeichnet, daß das Serum ein Antiserum ist.

7. Verfahren nach mindestens einem der Ansprüche 1 bis 5 dadurch gekennzeichnet, daß das Serum ein Standard- und/oder Kontroll-Serum ist.

8. Verwendung von cyclischen Carbonsäureamiden der allgemeinen Formel (I) zur Vermeidung von Trübungen und/oder Ausflockungen in Seren.

9. Serum zur Verwendung in der Diagnostik dadurch gekennzeichnet, daß es eine zur Verhinderung und/oder Beseitigung von Trübungen wirksame Menge einer oder mehrerer Verbindungen der Formel (I) enthält.

10. Serum gemäß Anspruch 9 dadurch gekennzeichnet, daß die Konzentration der Verbindungen der Formel (1) 0,2 - 30 Gew. % beträgt.

11. Serum gemäß Anspruch 10 dadurch gekennzeichnet, daß die Konzentration 1 - 10 Gew. % beträgt.

12. Verfahren nach mindestens einem der Ansprüche 1 - 7 dadurch gekennzeichnet, daß dem Serum noch andere bekannte Stabilisator substanzen, wie Natriumazid oder Antibiotika zugesetzt werden.

13. Verwendung der Seren nach mindestens einem der Ansprüche 9-11 als Standard- und/oder Kontrollserum in einem diagnostischen Verfahren.

14. Verwendung nach Anspruch 13, wobei das diagnostische Verfahren die Nephelometrie oder Turbidimetrie ist.

## Claims

1. A process for preparing clear sera which are stable over a long period and which are used in diagnostics, wherein a quantity of at least one compound of the formula (I) 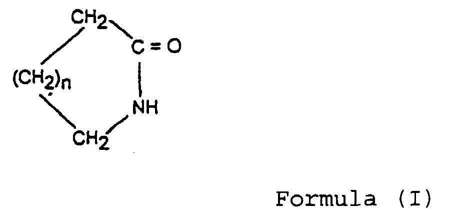 in which n is 0 - 8, preferably 1 - 4,
which is sufficient to prevent and/or eliminate a turbidity is added to the sera.

2. The process as claimed in claim 1, wherein compounds of the formula (I) are added to the sera in such quantities that the concentration of these compounds in the serum is from 0.2 to 30 % by weight.

3. The process as claimed in claims 1 and 2, wherein the compound of the formula (I) is butyrolactam.

4. The process as claimed in claims 1 to 3, wherein a mixture composed of at least 2 of the compounds of the formula (I) is added.

5. The process as claimed in claim 4, wherein butyrolactam and caprolactam are added.

6. The process as claimed in at least one of claims 1 to 5, wherein the serum is an antiserum.

7. The process as claimed in at least one of claims 1 to 5, wherein the serum is a standard serum and/or a control serum.

8. The use of cyclic carboxamides of the formula (I) for preventing turbidity and/or flocculation in sera.

9. A serum for use in diagnostics, which serum contains a quantity of one or more compounds of the formula (I) which is effective for preventing and/or eliminating turbidity.

10. The serum as claimed in claim 9, wherein the concentration of the compounds of the formula (I) is 0.2 - 30 % by weight.

11. The serum as claimed in claim 10, wherein the concentration is 1 - 10 % by weight.

12. The process as claimed in at least one of claims 1 - 7, wherein other known stabilizer substances, such as sodium azide or antibiotics, are also added to the serum.

13. The use of the sera as claimed in at least one of claims 9 - 11 as a standard serum and/or a control serum in a diagnostic method.

14. The use as claimed in claim 13, wherein the diagnostic method is nephelometry or turbidimetry.

## Revendications

1. Procédé de préparation de sérums limpides stables à long terme qui sont utilisés pour le diagnostic, caractérisé en ce que l'on ajoute aux sérums, en quantité suffisante pour empêcher et/ou éliminer une turbidité, un ou plusieurs composés de la formule (I) : 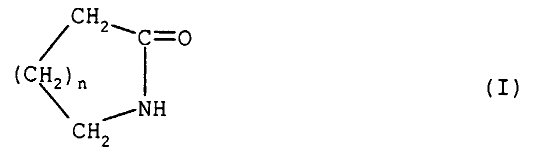 dans laquelle n = 0 à 8, de préférence n = 1 à 4.

2. Procédé selon la revendication 1, caractérisé en ce que l'on ajoute aux sérums des composés de la formule (I) en quantités telles que la concentration de ces composés dans le sérum est de 0,2 à 30 % en poids.

3. Procédé selon les revendications 1 et 2, caractérisé en ce que le composé de la formule (I) est le butyrolactame.

4. Procédé selon les revendications 1 à 3, caractérisé en ce que l'on ajoute un mélange d'au moins 2 des composés de la formule (I).

5. Procédé selon la revendication 4, caractérisé en ce que l'on ajoute du butyrolactame et du caprolactame.

6. Procédé selon l'une au moins des revendications 1 à 5, caractérisé en ce que le sérum est un antisérum.

7. Procédé selon l'une au moins des revendications 1 à 5, caractérisé en ce que le sérum est un sérum étalon et/ou de contrôle.

8. Utilisation de carboxylamides de la formule générale (I) pour empêcher des turbidités et/ou des floculations dans des sérums.

9. Sérum pour une utilisation dans le diagnostic, caractérisé en ce que qu'il contient une quantité d'un ou plusieurs composés de la formule (I) efficace pour empêcher et/ou éliminer des turbidités.

10. Sérum selon la revendication 9, caractérisé en ce que la concentration des composés de la formule (I) est de 0,2 à 30 % en poids.

11. Sérum selon la revendication 10, caractérisé en ce que la concentration est de 1 à 10 % en poids.

12. Procédé selon l'une au moins des revendications 1 à 7, caractérisé en ce que l'on ajoute en plus au sérum d'autres substances stabilisantes connues telles que l'azide de sodium ou des antibiotiques.

13. Utilisation des sérums selon l'une au moins des revendications 9 à 11 comme sérums étalons et/ou de contrôle dans un procédé diagnostique.

14. Utilisation selon la revendication 13, dans laquelle le procédé diagnostique est la néphélométrie ou la turbidimétrie.
